(19) Europäisches Patentamt
European Patent Office
Office européen des brevets

(11) EP 1 466 920 A1

(12) EUROPEAN PATENT APPLICATION

(43) Date of publication:
**13.10.2004 Bulletin 2004/42**

(51) Int Cl.[7]: **C07J 3/00**, C07J 31/00

(21) Application number: **03007756.4**

(22) Date of filing: **04.04.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Alpharma APS**
**2300 Copenhagen S (DK)**

(72) Inventors:
- **Loevli, Trond**
  **0464 Oslo (NO)**
- **Nygard, Anne Mette**
  **0363 Oslo (NO)**
- **Reitstoen, Bjoern**
  **0374 Oslo (NO)**
- **Fivelstad, Magny**
  **0855 Oslo (NO)**

# (54) Process for the preparation of steroidal 17 beta-carbothioates

(57) A novel method for the conversion of steroidal 17β-carboxylic acids to carbothioates such as fluticasone propionate via novel *in situ* generated 17β-carboxy imidazolyl- or succinimidyl esters.

EP 1 466 920 A1

## Description

## Technical Field

The present invention relates to a novel process for the conversion of steroidal 17β-carboxylic acids to carbothioates such as fluticasone propionate via novel *in situ* generated 17β-carboxy imidazolyl- or succinimidyl esters.

## Background of the Invention

In peptide chemistry the need to activate carboxylic acids in order to facilitate peptide couplings has been recognized for decades *(Handbook of Reagents for Organic Synthesis, Activating Agents and Protecting Groups,* ed. A. J. Pearson and W.R. Roush, John Wiley & Sons, 1999). The initial coupling agent, e.g. a carbodiimide, is often used in conjunction with a coupling enhancer. The use of coupling enhancers is said to improve reaction rates and minimize side reactions.

Within the field of steroids the use of coupling agents have also found widespread use, especially in the preparation of carbothioic acids and esters of the androstane series (e.g. US4188385, US4198403, US4335121, US4578221, WO02088167, WO02100879, WO0212265, WO0212266). However, in (the majority of) these cases coupling enhancers were not used, and as a result some of the activated intermediates suffered the disadvantage of being prone to competing reactions, e.g. hydrolysis, resulting in a reduced yield of the final product. In US6197761, Glaxo made use of coupling enhancers. In example 16, a novel oxo-tetra-hydrofuranoyl amide was prepared by activating the androstane 17β-carboxylic acid with 1-ethyl-3-(3-dimethyl-aminopropyl) carbodiimide hydrochloride (EDC) in conjunction with 1-hydroxybenzotriazole (HOBt).

In the preparation of 17β-carbothioic acids, sulphur has generally been introduced under anhydrous conditions; either by the use of hydrogensulfide (US4335121, US4578221) or by employing the salt of hydrogensulfide, generated *in situ* from the reaction between sodium hydride and hydrogensulfide (US4188385, US4335121, US4578221).

In US2002/0133032 Abbott claims a process towards fluticasone propionate starting from flumethasone. The process consists of 5 steps, of which the final two can be performed as a one-pot reaction, and the product is obtained in an overall yield of 52 %. The chemistry involved has been carefully picked from prior art such as US4335121 and Phillips *et al.,* J. Med. Chem., 1994, 37, 3717-3729, both of Glaxo. In the above mentioned patent, Abbott also claims that a 6α-chloro-9α-fluoro-impurity present in commercial grade flumethasone necessitates an elaborate purification of flumethasone prior to use. However, new methods for the preparation of flumethasone (US2002/0062021) makes it possible to avoid this added complication.

In the preparation of fluticasone propionate, the introduction of the $CH_2F$-moiety has been accomplished by the reaction of the carbothioic metal salt with a dihalomethane-derivative, preferably a fluorohalomethane. Glaxo first made use of bromochloro-methane, followed by halogen exchanges (US4335121). They later moved on to bromofluoromethane (WO02088167, WO02100879, WO0212265, WO0212266), first mentioned by Phillips *et al.* (J. Med. Chem., 1994, 37, 3717-3729). The Israeli company Chemagis (IL109656) utilised both bromo- and chlorofluoromethane. Chlorofluoromethane was later used by Abbott as well (US2002/0133032).

## Summary of invention:

The problem to be solved by the present invention is to provide a new method for preparing a steroidal carbothioate such as fluticasone propionate where a number of the relevant method steps may be made as a so-called continuous one-pot synthesis. This denotes a method where relevant steps may be made *in situ* without e.g. change of solvent or separate purification steps of synthesized intermediates.

The solution is based on that the present inventors have identified that by including the use of a suitable novel *in situ* generated 17β-carboxy imidazolyl- or succinimidyl ester as intermediate it was possible to develop such a method. This intermediate is defined as formula (III) herein. It was found that this intermediate has an increased threshold against competing hydrolysis reactions, which therefore provides the possibility of making relevant method steps as a continuous one-pot synthesis. The reduced level of hydrolysis further raises the efficiency regarding the formation of the end steroidal carbothioate product. Preferred steroidal carbothioate end product is defined as formula (I) herein.

Where $R_{10}$ of formula (I) is a fluoromethyl group this formula (I) represents fluticasone propionate.

Accordingly, a first aspect of the invention relates to a compound consisting of the formula (III)

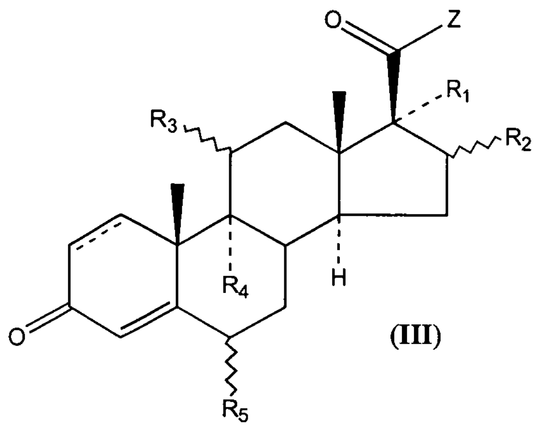

(III)

wherein $R_1$ represents a hydrogen atom, a hydroxy group in the α-configuration, a group $OCOR_6$, where $R_6$ is a $C_{1-6}$ alkyl group or a 5-6 membered heterocyclic ring containing either oxygen, nitrogen or sulfur; $R_2$ represents a hydrogen atom, a hydroxy group in the α-configuration, a $C_{1-6}$ alkyl group which may be in either the α- or β-configuration, a methylene group or $R_1$ and $R_2$ together represent

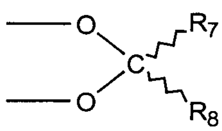

where $R_7$ and $R_8$ are the same or different and each represent a hydrogen atom or a $C_{1-6}$ alkyl group; $R_3$ represent a hydroxy or an optionally protected hydroxy group in either the α- or β-configuration or an oxo group; $R_4$ represents a hydrogen atom or a halide or $R_3$ and $R_4$ together represent a carbon-carbon bond or an epoxy group in the β-configuration; $R_5$ represents a hydrogen atom or a halide in either the α- or β-configuration; and Z represent a imidazolyl radical of formula (A) or a succinimidyl radical of formula (B)

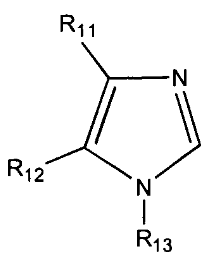

(A)

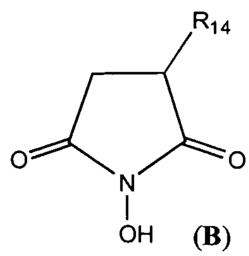

(B)

wherein $R_{11}$ and $R_{12}$ represent hydrogen atoms or cyano groups (C≡N); $R_{13}$ represent a hydrogen atom or a methyl group; and $R_{14}$ represent a hydrogen atom or a salt of a sulfonic acid.

The symbol $\overline{-----}$ in the 1,2-position represent a single or a carbon-carbon double bond.

As explained above, this intermediate of formula (III) is very suitable for use in a method for preparing a steroidal carbothioate by converting a steroidal carboxylic acid to the steroidal carbothioate.

Accordingly, a second aspect of the invention relates to use of a compound of the first aspect of the invention as an intermediate in a method for preparing a steroidal carbothioate by converting a steroidal carboxylic acid to the steroidal carbothioate.

As explained above, an advantage of the herein described method relates to the possibility of making relevant method steps *in situ.*

Accordingly, a third aspect of the invention relates to a method for preparing a steroidal carbothioate of formula (I)

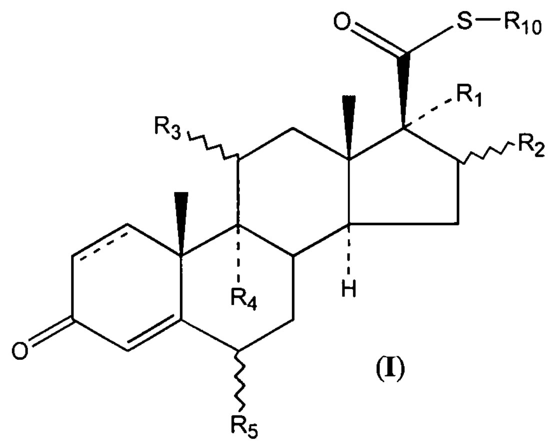

(I)

wherein $R_1$ represents a hydrogen atom, a hydroxy group in the α-configuration, a group $OCOR_6$, where $R_6$ is a $C_{1-6}$ alkyl group or a 5-6 membered heterocyclic ring containing either oxygen, nitrogen or sulfur; $R_2$ represents a hydrogen atom, a hydroxy group in the α-configuration, a $C_{1-6}$ alkyl group which may be in either the α- or β-configuration, a methylene group or $R_1$ and $R_2$ together represent

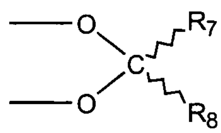

where $R_7$ and $R_8$ are the same or different and each represent a hydrogen atom or a $C_{1-6}$ alkyl group; $R_3$ represent a hydroxy or an optionally protected hydroxy group in either the α- or β-configuration or an oxo group; $R_4$ represents a hydrogen atom or a halide or $R_3$ and $R_4$ together represent a carbon-carbon bond or an epoxy group in the β-configuration; $R_5$ represents a hydrogen atom or a halide in either the α- or β-configuration; and $R_{10}$ represent a fluoro-, chloro-, bromomethyl group or a 2'-fluoroethyl group, the method comprising:

A) reacting a steroidal carboxylic acid of formula (II)

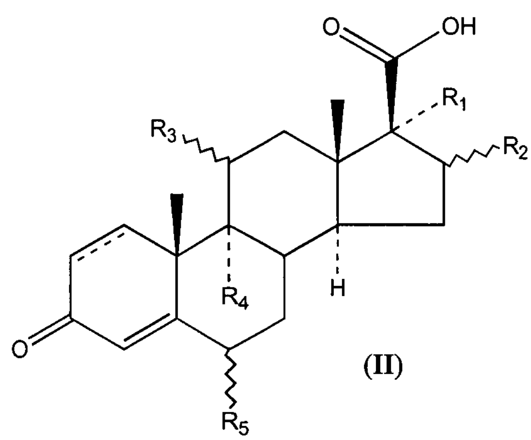

(II)

with a coupling enhancer of formula (A) or formula (B)

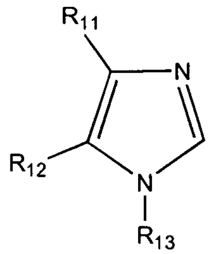 (A) 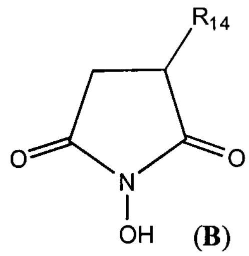 (B)

wherein $R_{11}$ and $R_{12}$ represent hydrogen atoms or cyano groups (C≡N); $R_{13}$ represent a hydrogen atom or a methyl group; and $R_{14}$ represent a hydrogen atom or a salt of a sulfonic acid;

B) reacting *the in situ* generated imidazolyl- or succinimidyl ester from step A) with a nucleophilic agent comprising sulfide; and

C) reacting the *in situ* product from step B) with an electrophilic agent.

Among others due to the stability of the intermediate of formula (III) against competing hydrolysis reactions, further advantages of the method as described herein relates to that there is no need to work under anhydrous conditions. This enables one to avoid the use of hydrogensulfide gas, allowing instead the use of hydrosulfide salts, either as solids with crystal water or as solutions of the desired sulfide salt in water. Further it sets the stage for *an in situ* process where three of the five steps may be performed in one-pot. Further e.g. fluticasone propionate may be obtained in an overall yield of 75 % (see working example 3 herein).

Embodiments of the present invention are described below, by way of examples only.

## Detailed description of the invention

The method as described herein is in a preferred embodiment for the conversion of androstane 17β-carboxylic acids to 17β-carbothioates such as e.g. fluticasone propionate via *novel in situ* activated 17β-carboxylic acid intermediates. In a preferred embodiment of the method the 17β-carbothioic esters are prepared by reacting an acid of formula (II) with a coupling agent in conjunction with a coupling enhancer. The terms "coupling agent" and "coupling enhancer" are herein used to refer to chemical reagents that facilitate the formation of a reactive intermediate of formula (III) where Z represent compounds of formula (A) or formula (B). Such intermediates may be formed between androstane 17β-carboxylic acids and 1-ethyl-3-(3-dimethyl-aminopropyl) carbodiimide hydrochloride (EDC) (a preferred example of a coupling agent) in combination with suitable coupling enhancers.

Suitable coupling enhancers as used herein can for example be represented by the structures of formula (A) and formula (B) as described above.

The coupling agent and the optional coupling enhancers are preferably water-soluble so the reactions could be effected in aqueous solutions if preferred.

In formula (B) preferably at least one of $R_{11}$ and $R_{12}$ is a cyano group (C≡N). Further, $R_{13}$ may preferably be a hydrogen atom. Preferred examples are wherein the formula (A) is NMI (N-methylimidazole) or DCI (4,5-dicyano-imidazole) or wherein the formula (B) is NHS (N-hydroxysuccinimide).

According to one embodiment of the present invention, compounds of formula (III) as defined hereinbefore may be prepared by treating a compound of formula (II) with EDC in combination with a compound of formula (A). The resulting 17β-carboxy imidazolyl ester, which may be isolated if required, is treated with a nucleophilic agent (sulfide source) that displaces the active ester group to form a compound of formula (IV), wherein $R_9$ represent a hydrogen atom or a salt, a methyl, ethyl or isopropyl group. Compounds of formula (IV) may be isolated if necessary. Subsequent addition of an electrophilic agent leads to the formation of compounds of formula (I) as depicted in scheme 1, wherein $R_{10}$ is a fluoro-, chloro-, bromomethyl group or a 2'-fluoroethyl group.

Preferably the nucleophilic agent is an alkoxide salt, a thioalkoxide salt, or more preferably a hydrated sulfide salt.

Preferably the electrophilic agent is chlorofluoromethane or more preferably bromofluoromethane.

In a preferred embodiment of the present method, compounds of formula (III) as defined hereinbefore may be prepared by treating a compound of formula (II) with EDC in combination with a compound of formula (B). The resulting 17β-carboxy succinimidyl ester which may be isolated if required is treated with a nucleophilic agent that displaces the active ester group to form a compound of formula (IV) as defined hereinbefore. Subsequent addition of a electrophilic agent conclude the synthesis towards compounds of formula (I) as depicted in scheme 1, wherein $R_{10}$

is a fluoro-, chloro-, bromomethyl group or a 2'-fluoroethyl group. The formation of compounds of formula (I) may take place in a one-pot reaction starting with compounds of formula (II) without isolation of the intermediates of formula (III) and (IV) if e.g. sodium hydrosulfide hydrate is used as the nucleophilic agent.

The use of a coupling enhancer in combination with a carbodiimide forms an active ester intermediate of formula (III) with increased stability and reactivity. These intermediates have an increased threshold against competing hydrolysis reactions. The reduced level of hydrolysis raises the efficiency regarding the formation of products of formula (I) (scheme 1, see below) where $R_{10}$ is defined as hereinbefore.

We have surprisingly found that the use of these novel 17β-carboxy succinimidyl- and 17β-carboxy imidazolyl esters of formula (III) reduce the cycle time, increase the yield and enhance the purity of the final product of formula (I).

Compound preparation

The method of the disclosed invention will be better understood when related to the following synthetic scheme, which illustrates a preferred embodiment of the method as described herein. Moreover, persons skilled in the art will be aware of variations of, and alternatives to the method described hereinafter. The provided examples will demonstrate the synthesis and allow compounds defined by formula (I) to be obtained.

**Scheme 1**

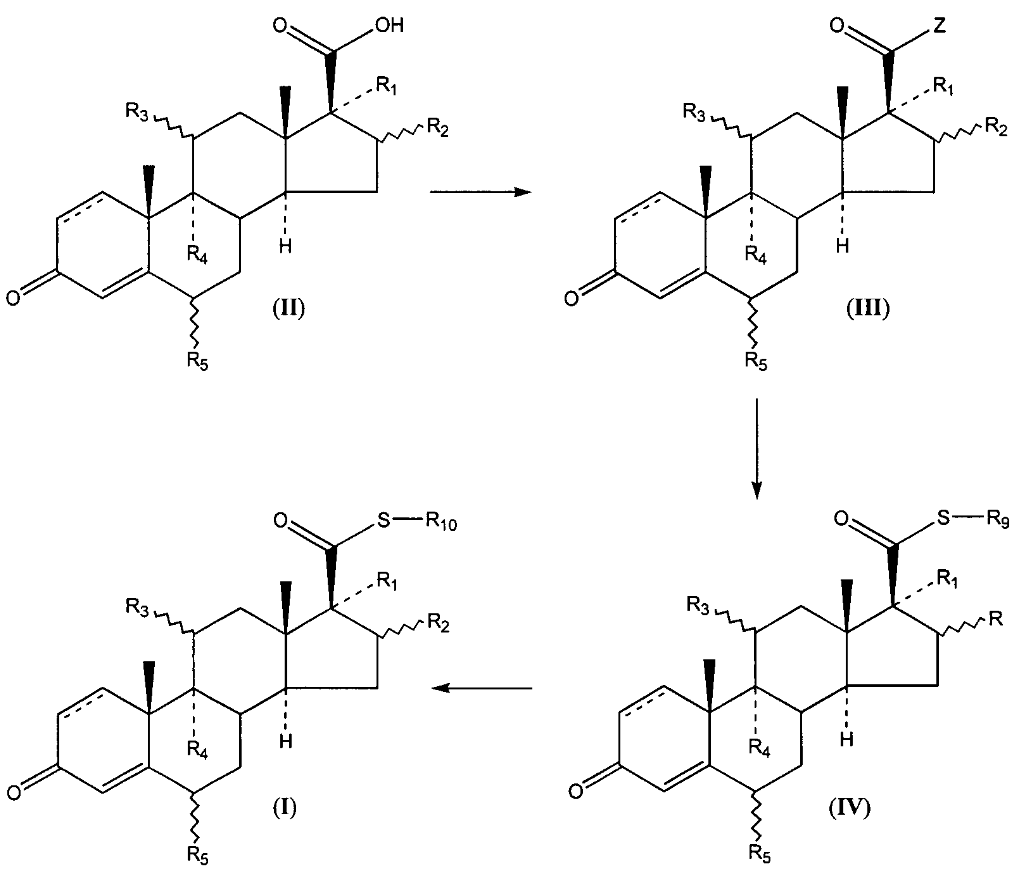

The androstane 17β-carboxylic acid starting materials employed in the present invention may be readily prepared by any means known in the art. Compounds of formula (II) can be prepared by oxidation of a suitable 21-hydroxy-20-keto pregnane of formula (V) as taught in e.g. US3636010 (example 1).

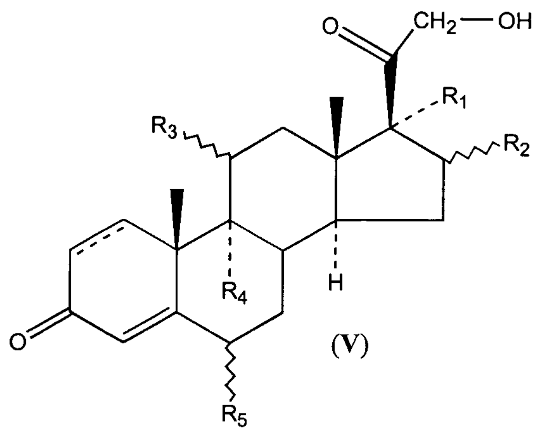

(V)

Compounds of formula (V) which are commercially available include e.g. budesonide, desonide, triamcinolone acetonide, fluocinolone acetonide, betamethasone, dexamethasone, prednisolone, flumethasone, beclomethasone, icomethasone, diflorasone, hydrocortisone and fludrocortisone.

The method as described herein will now be described in detail in connection with other particular preferred embodiments of scheme 1. Thus, the following and not limiting examples will illustrate an especially preferred methodology. The examples are presented to provide what is believed to be the most useful and readily understood description of the procedures and conceptual aspects of the method as described herein.

## Examples

General

MS (ESI) refer to mass spectra using electrospray ionisation techniques. Melting points were obtained using a Mettler FP81 MBC cell and are uncorrected. The purity of the products was determined by RP18-HPLC analysis using UV-detection at 239 nm. Abbreviations; DMF (N,N-dimethylformamide), DMA (N,N-dimethylacetamide), THF (tetrahydrofurane), EDC (1-ethyl-3-(3-dimethyl-aminopropyl)carbodiimide hydrochloride), NHS (N-hydroxysuccinimide), NMI (N-methylimidazole) and DCI (4,5-dicyano-imidazole).

Example 1

6α,9α-difluoro-11β, 17α-dihydroxy-16α-methyl-3-oxoandrosta-1,4-diene-17β-carboxylic acid

6α,9α-difluoro-11 β,17α-21-trihydroxy-16α-methyl-pregna-1,4-diene-3,20-dione (flumetasone) (5.25 g, 12.78 mmol) was dissolved in THF (25 ml) at 0 °C. To the stirred solution, periodic acid (2.0 eq., 5.83 g in 12 ml $H_2O$) was added dropwise. The reaction flask was covered in aluminium foil after 75 minutes and left stirring at room temperature. The reaction was quenched after 4.5 hours by addition of water (120 ml) and left in a refrigerator over-night. The resulting precipitate was filtered, washed with water until the filtrate was neutral to pH-paper and dried under reduced pressure. The title compound was obtained as a white solid (4.96 g, 98%), with a purity of >99% by HPLC.

Example 2

6α,9α-difluoro-11β-hydroxy-16α-methyl-3-oxo-17α-propionyloxyandrosta-1,4-diene-17β-carboxylic acid

A suspension of 6α,9α-difluoro-11β,17α-dihydroxy-16α-methyl-3-oxoandrosta-1,4-diene-17β-carboxylic acid (4.11 g, 10.38 mmol) in acetone (30 ml) was added $Et_3N$ (3.5 eq., 3.7 ml) under $N_2$ and stirring. The resulting solution was cooled to 0 °C and propionyl chloride (4.5 eq., 4.3 ml) was added dropwise to a white suspension. After 85 minutes $Et_2NH$ (10.0 eq., 7.6 ml) was added and the resulting mixture was left stirring for 55 minutes at 0 °C, before the solvent was removed under reduced pressure. The resulting solid was dissolved in water (20 ml), and treated with 1N HCl to pH 2. The white precipitate thus formed was filtered, washed with ice cold water and dried under reduced pressure at 60°C. The title compound was obtained as a white solid (4.64 g, 99%) with a purity of 99% by HPLC.

Example 3

S-fluoromethyl 6α,9α-difluoro-11β-hydroxy-16α-methyl-3-oxo-17α-propionyloxyandrosta-1,4-diene-17β-carbothioate (fluticasone propionate)

A flask charged with 6α,9α-difluoro-11β-hydroxy-16α-methyl-3-oxo-17α-propionyloxy-androsta-1,4-diene-17β-carboxylic acid (0.30 g, 0.66 mmol) in DMA (30 ml) at ~50°C was added EDC (3.0 eq., 0.38 g) and NHS (3.1 eq., 0.24 g) and left stirring until the reaction was complete, as monitored by HPLC analysis. The oil bath was turned off and sodium hydrosulfide hydrate (20.0 eq., 0.74 g) was added in one portion at ~35 °C. The addition immediately resulted in a strongly blue coloured reaction mixture. After 15 minutes the reaction was complete and gaseous bromofluormethane was added at roomtemperature. The initial blue colour gradually changed to pale yellow during the addition of bromofluoromethane. On completion of the reaction, after 15 minutes, as monitored by HPLC analysis, sodiumcarbonate (25 ml, 10% in water) followed by water (10 ml) was added to the cooled reaction mixture. The solid thus formed was filtered, dissolved in methanol (40 ml), heated to reflux and filtered. Temperated water (40 ml) was slowly added to the warm filtrate. The beginning suspension was left at roomtemperature for two hours before cooling in a refrigerator. The resulting precipitate was collected by filtration, washed with water (40 ml) and dried under reduced pressure. The title compound was obtained as a white amorphous solid (0.25 g, 75%) with a melting point of 267 °C and a purity of>98% by HPLC. Spiking of the product sample with the British Pharmacopeia Standard (melting point: 265 °C) of fluticasone propionate, showed one peak by HPLC analysis.

Example 4

6α,9α-difluoro-11β-hydroxy-16α-methyl-3-oxo-17α-propionyloxyandrosta-1,4-diene-17β-carbothioic acid

A flask charged with 6α,9α-difluoro-11β-hydroxy-16α-methyl-3-oxo-17α-propionyl-oxyandrosta-1,4-diene-17β-carboxylic acid (0.30 g, 0.66 mmol) in DMA (30 ml) was heated to ~50 °C. EDC (3.0 eq., 0.38 g) and NMI (3.1 eq., 162 μl) was added and the reaction mixture was left stirring until the reaction was complete, as monitored by HPLC analysis. The oil bath was turned off and sodium hydrosulfide hydrate (20.0 eq., 0.74 g) was added in one portion at ~35 °C. The addition immediately resulted in a strongly blue coloured reaction mixture with some precipitation. After 15 minutes the reaction was complete and crushed ice followed by 2N HCl (10 ml) to pH ~2 was added and the flask was left in a refrigerator. During the addition of HCl, the initial bluish reaction mixture gradually became colorless. The solid thus formed was filtered and a minimum amount of acetone following temperated water was addded to a beginning crystallisation. The refrigerated suspension was filtered, washed with water (30 ml) until the filtrate was neutral to pH-paper and dried under reduced pressure. The title compound was obtained as a white amorphous solid (0.10 g, 32%) with a purity of 98% by HPLC.

Example 5

S-methyl 6α,9α-difluoro-11β-hydroxy-16α-methyl-3-oxo-17α-propionyloxyandrosta-1,4-diene-17β-carbothioate

6α, 9α-difluoro-11 β-hydroxy-16α-methyl-3-oxo-17α-propionyloxyandrosta-1,4-diene-17β-carboxylic acid (0.20 g, 0.43 mmol) was dissolved in DMF (10 ml). The reaction flask was immersed in an oil-bath and the temperature was raised to ~40 °C. EDC (~2 eq., 0.15 g) and NHS (~5 eq., 0.26 g) was added and the reaction mixture was left stirring at 40 °C for 19 hours. The temperature was raised to 50 °C and another portion of EDC (~4 eq., 0.29 g) was added. The mixture was left stirring at 50 °C for 3 hours. The flask was removed from the oil-bath and the reaction mixture was diluted by the addition of DMF (5 ml). Aqueous NaSMe (1.0 ml, 21% solution in water) was added in portions over a period of 30 minutes. The reaction mixture turned into a pale pink suspension. The reaction was quenched by the addition crushed ice and aqueous HCl (12 ml, 10%). The white precipitate was filtered, washed with water and dried. The crude product was dissolved in a minimum amount of acetone and water was added until a beginning precipitation. The resulting solid was collected by filtration, washed with water and dried under reduced pressure to yield the title compound as a white solid (0.11 g, 51 %) with a purity of 90% by HPLC.

Example 6 (theoretical experiment)

S-methyl 6α,9α-difluoro-11β-hydroxy-16α-methyl-3-oxo-17α-propionyloxyandrosta-1,4-diene-17β-carbothioate

A flask charged with 6α,9α-difluoro-11β-hydroxy-16α-methyl-3-oxo-17α-propionyl-oxyandrosta-1,4-diene-17β-carboxylic acid (0.30 g, 0.66 mmol) in DMA (30 ml) was heated to ~50 °C. EDC (3.0 eq., 0.38 g) and NMI (3.1

eq., 162 µl) was added and left stirring until the reaction was complete, as monitored by HPLC analysis. The oil bath was turned off and and aqueous NaSMe (1.5 ml, 21% solution in water) was added in one portion at ~35 °C. The reaction was quenched by the addition of chrused ice and aqueous HCl (12 ml, 10%). The white precipitate was filtered, washed with water and dried. The crude product was dissolved in a minimum amount of acetone and added water. The resulting solid was collected by filtration washed with water and dried under reduced pressure to yield the title compound as a white solid (x g, x%) with a purity of x% by HPLC.

Example 7 (theoretical experiment)

S-fluoromethyl 6α,9α-difluoro-11β-hydroxy-16α-methyl-3-oxo-17α-propionyloxyandrosta-1,4-diene-17β-carbothioate (fluticasone propionate)

A flask charged with 6α,9α-difluoro-11β-hydroxy-16α-methyl-3-oxo-17α-propionyl-oxyandrosta-1,4-diene-17β-carboxylic acid (0.30 g, 0.66 mmol) in DMA (30 ml) at ~50 °C was added EDC (3.0 eq., 0.38 g) and DCI (3.1 eq.) and left stirring until the reaction was complete, as monitored by HPLC analysis. The oil bath was turned off and sodium hydrosulfide hydrate (20.0 eq., 0.74 g) was added in one portion at ~35 °C. After x minutes the reaction was complete and gaseous bromofluormethane was added at roomtemperature. On completion of the reaction, as monitored by HPLC analysis, sodiumcarbonate (25 ml, 10% in water) followed by water (10 ml) was added to the cooled reaction mixture. The solid thus formed was filtered, dissolved in methanol (40 ml), heated to reflux and filtered. Temperated water (40 ml) was slowly added to the warm filtrate. The beginning suspension was left at roomtemperature for two hours before cooling in a refrigerator. The resulting precipitate was collected by filtration, washed with water (40 ml) and dried under reduced pressure. The title compound was obtained as a white amorphous solid (x g, x%) with a melting point of x °C and a purity of x% by HPLC.

## Invention described in a claim representation

The aspects and preferred embodiments of the present invention may be described in form of claims. Such a representation is given immediately below.

**1**. A compound consisting of the formula (III)

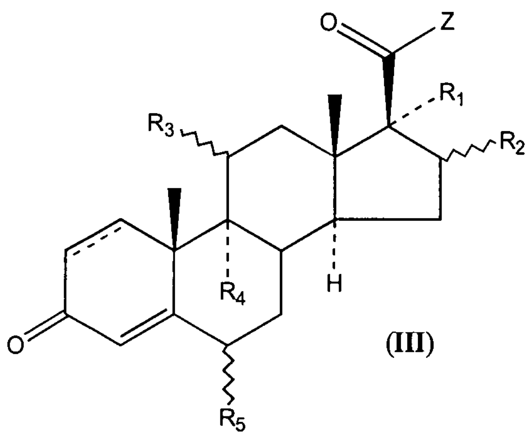

(III)

wherein $R_1$ represents a hydrogen atom, a hydroxy group in the α-configuration, a group $OCOR_6$, where $R_6$ is a $C_{1-6}$ alkyl group or a 5-6 membered heterocyclic ring containing either oxygen, nitrogen or sulfur; $R_2$ represents a hydrogen atom, a hydroxy group in the α-configuration, a $C_{1-6}$ alkyl group which may be in either the α- or β-configuration, a methylene group or $R_1$ and $R_2$ together represent

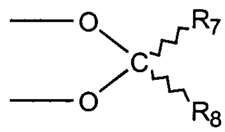

where $R_7$ and $R_8$ are the same or different and each represent a hydrogen atom or a $C_{1-6}$ alkyl group; $R_3$ represent a hydroxy or an optionally protected hydroxy group in either the α- or β-configuration or an oxo group; $R_4$ represents a hydrogen atom or a halide or $R_3$ and $R_4$ together represent a carbon-carbon bond or an epoxy group in the β-configuration; $R_5$ represents a hydrogen atom or a halide in either the α- or β-configuration; and Z represent a imidazolyl radical of formula (A) or a succinimidyl radical of formula (B)

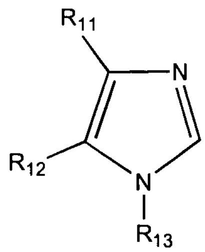
(A)

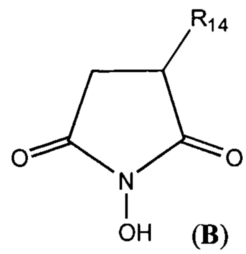
(B)

wherein $R_{11}$ and $R_{12}$ represent hydrogen atoms or cyano groups (C≡N); $R_{13}$ represent a hydrogen atom or a methyl group; and $R_{14}$ represent a hydrogen atom or a salt of a sulfonic acid.

**2**. The compound of claim 1, wherein at least one of $R_{11}$ and $R_{12}$ is a cyano group (C≡N).

**3**. The compound of claims 1 or 2, wherein $R_{13}$ is a hydrogen atom.

**4**. The compound of claim 1, wherein the formula (A) is NMI (N-methylimidazole) or DCI (4,5-dicyano-imidazole) or wherein the formula (B) is NHS (N-hydroxysuccinimide).

**5**. Use of a compound of any of the claims 1 to 4 as an intermediate in a method for preparing a steroidal carbothioate by converting a steroidal carboxylic acid to the steroidal carbothioate.

**6**. A method for preparing a steroidal carbothioate of formula (I)

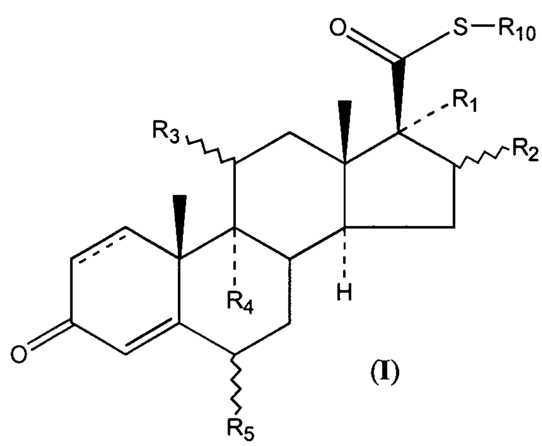
(I)

wherein $R_1$ represents a hydrogen atom, a hydroxy group in the α-configuration, a group $OCOR_6$, where $R_6$ is a $C_{1-6}$ alkyl group or a 5-6 membered heterocyclic ring containing either oxygen, nitrogen or sulfur; $R_2$ represents

a hydrogen atom, a hydroxy group in the α-configuration, a $C_{1-6}$ alkyl group which may be in either the α- or β-configuration, a methylene group or $R_1$ and $R_2$ together represent

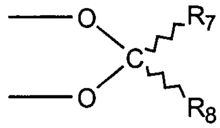

where $R_7$ and $R_8$ are the same or different and each represent a hydrogen atom or a $C_{1-6}$ alkyl group; $R_3$ represent a hydroxy or an optionally protected hydroxy group in either the α- or β-configuration or an oxo group; $R_4$ represents a hydrogen atom or a halide or $R_3$ and $R_4$ together represent a carbon-carbon bond or an epoxy group in the β-configuration; $R_5$ represents a hydrogen atom or a halide in either the α- or β-configuration; and $R_{10}$ represent a fluoro-, chloro-, bromomethyl group or a 2'-fluoroethyl group, the method comprising:

A) reacting a steroidal carboxylic acid of formula (II)

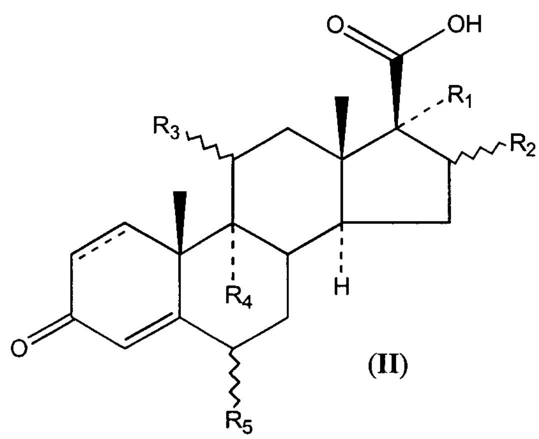

(II)

with a coupling enhancer of formula (A) or formula (B)

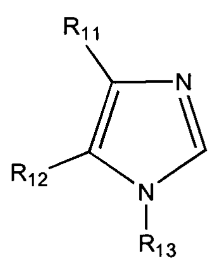

(A)

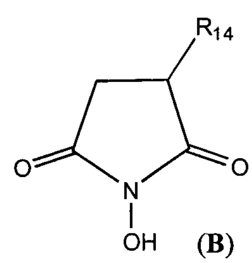

(B)

wherein $R_{11}$ and $R_{12}$ represent hydrogen atoms or cyano groups (C≡N); $R_{13}$ represent a hydrogen atom or a methyl group; and $R_{14}$ represent a hydrogen atom or a salt of a sulfonic acid;
B) reacting *the in situ* generated imidazolyl- or succinimidyl ester from step A) with a nucleophilic agent comprising sulfide; and
C) reacting *the in situ* product from step B) with an electrophilic agent.

**7**. The method of claim 6, wherein at least one of $R_{11}$ and $R_{12}$ is a cyano group (C≡N).

**8**. The method of claims 6 or 7, wherein $R_{13}$ is a hydrogen atom.

**9**. The method of claim 6, wherein the formula (A) is NMI (N-methylimidazole) or DCI (4,5-dicyano-imidazole) or wherein the formula (B) is NHS (N-hydroxysuccinimide).

**10.** The method of any of the claims 6-9, wherein step A) is reacting the steroidal carboxylic acid with a coupling agent and the coupling enhancer.

**11.** The method of claim 10, wherein the coupling enhancer is subsequently added after the coupling agent.

**12.** The method of claim 10, wherein the steroidal carboxylic acid is added to a mixture of the coupling agent and the coupling enhancer.

**13.** The method of claim 10, wherein a mixture of the coupling agent and the coupling enhancer is added to a steroidal carboxylic acid.

**14.** The method of any of the claims 6-13, wherein the nucleophilic agent is an alkoxide salt, a thioalkoxide salt, or a hydrated sulfide salt.

**15.** The method of any of the claims 6-14, wherein the nucleophilic agent is dissolved in a suitable solvent prior to addition to the reaction mixture.

**16.** The method of any of the claims 6-15, wherein the nucleophilic agent is a salt, or a solution of the salt in water and/or an organic solvent or a combination thereof.

**17.** The method of any of the claims 6-16 conducted as a continuous method.

**18.** The method of claim 17, wherein step A), B) and C) are conducted in a one-pot synthesis without solvent changes and is conducted at room or elevated temperature.

**19.** The method of any of the claims 6-18, wherein the electrophilic agent is chlorofluoromethane or preferably bromofluoromethane.

**20.** The method of any of the claims 6-19, wherein the steroidal carboxylic acid is added to a mixture of the coupling agent and the coupling enhancer in a polar aprotic solvent preferably DMF or DMA at elevated temperature.

**21.** The method of any of the claims 6-20, wherein the coupling agent is 1-ethyl-3-(3-dimethyl-aminopropyl) carbodiimide hydrochloride (EDC).

**22.** The method of any of the claims 6-21, wherein the method is a method where an androstane 17β-carboxylic acid is converted to a steroidal carbothioate and formula (II) is an androstane of formula (II).

**23.** The method of any of the claims 6-22, wherein step B) provide a compound of formula (IV), wherein $R_9$ is a sodium cation of the 17β-carbothioic acid

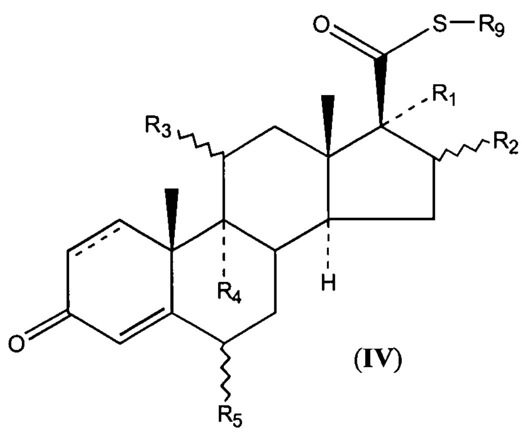

**(IV)**

**24.** The method of the claims 6-23, wherein the nucleophilic agent is sodium hydrosulfide hydrate.

**25.** The method of any of the claims 6-24, wherein step C) is reacting the *in situ* product from step B) with bromofluoromethane to form a compound of formula (I) wherein $R_{10}$ is a fluoromethyl group.

**26.** The method of any of the claims 6-25, wherein $R_{10}$ is a fluoromethyl group.

**A separate aspect of the invention**

Without being limited to theory, it is believed that the method as described herein may be used generally to as a method for preparing a steroidal carbothioate by converting a steroidal carboxylic acid to a carbothioate.

Accordingly a separate aspect of the invention relates to a method for preparing a steroidal carbothioate by converting a steroidal carboxylic acid to a carbothioate, the method comprising:

A) reacting a steroidal carboxylic acid with a coupling agent and a coupling enhancer of formula (A) or formula (B),

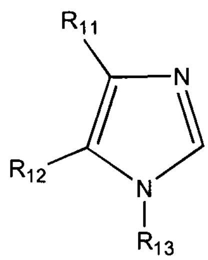
(A)

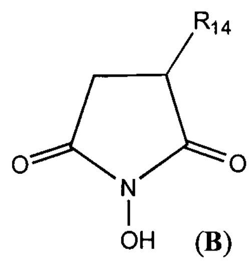
(B)

wherein $R_{11}$ and $R_{12}$ represent hydrogen atoms or a cyano groups (C≡N); $R_{13}$ represent a hydrogen atom or a methyl group and $R_{14}$ represent a hydrogen atom or a salt of a sulfonic acid;
B) reacting the *in situ* generated imidazolyl- or succinimidyl ester from step A) with a nucleophilic agent comprising sulfide; and
C) reacting the *in situ* product from step B) with an electrophilic agent.

All other aspects and preferred embodiments as described herein also applies to this separate aspect of the invention.

**Claims**

**1.** A compound consisting of the formula (III)

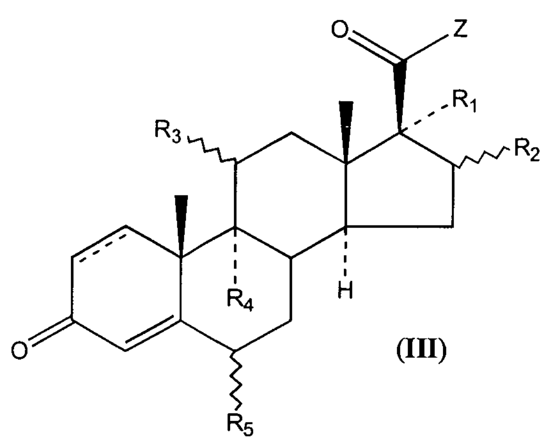
(III)

wherein $R_1$ represents a hydrogen atom, a hydroxy group in the α-configuration, a group $OCOR_6$, where $R_6$ is a $C_{1-6}$ alkyl group or a 5-6 membered heterocyclic ring containing either oxygen, nitrogen or sulfur; $R_2$ represents a hydrogen atom, a hydroxy group in the α-configuration, a $C_{1-6}$ alkyl group which may be in either the α- or β-configuration, a methylene group or $R_1$ and $R_2$ together represent

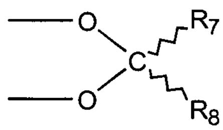

where $R_7$ and $R_8$ are the same or different and each represent a hydrogen atom or a $C_{1-6}$ alkyl group; $R_3$ represent a hydroxy or an optionally protected hydroxy group in either the α- or β-configuration or an oxo group; $R_4$ represents a hydrogen atom or a halide or $R_3$ and $R_4$ together represent a carbon-carbon bond or an epoxy group in the β-configuration; $R_5$ represents a hydrogen atom or a halide in either the α- or β-configuration; and Z represent a imidazolyl radical of formula (A) or a succinimidyl radical of formula (B)

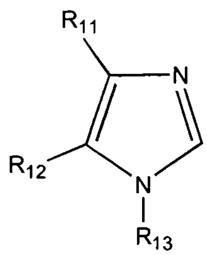
(A)

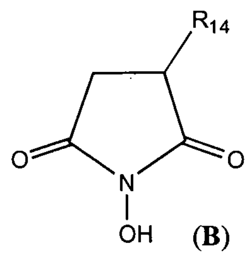
(B)

wherein $R_{11}$ and $R_{12}$ represent hydrogen atoms or cyano groups (C≡N); $R_{13}$ represent a hydrogen atom or a methyl group; and $R_{14}$ represent a hydrogen atom or a salt of a sulfonic acid.

2. The compound of claim 1, wherein the formula (A) is NMI (N-methylimidazole) or DCI (4,5-dicyano-imidazole) or wherein the formula (B) is NHS (N-hydroxysuccinimide).

3. Use of a compound of any of the claims 1 to 2 as an intermediate in a method for preparing a steroidal carbothioate by converting a steroidal carboxylic acid to the steroidal carbothioate.

4. A method for preparing a steroidal carbothioate of formula (I)

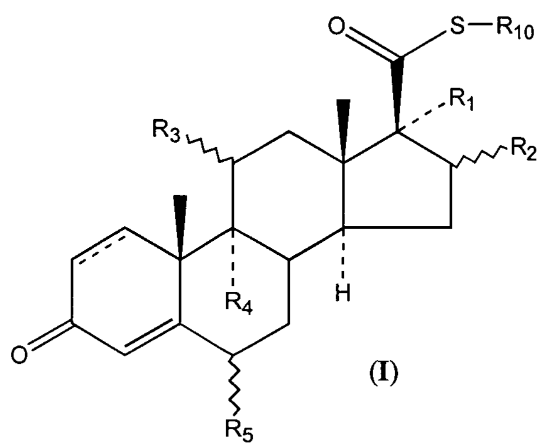
(I)

wherein $R_1$ represents a hydrogen atom, a hydroxy group in the α-configuration, a group $OCOR_6$, where $R_6$ is a $C_{1-6}$ alkyl group or a 5-6 membered heterocyclic ring containing either oxygen, nitrogen or sulfur; $R_2$ represents a hydrogen atom, a hydroxy group in the α-configuration, a $C_{1-6}$ alkyl group which may be in either the α- or β-configuration, a methylene group or $R_1$ and $R_2$ together represent

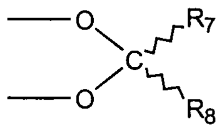

where $R_7$ and $R_8$ are the same or different and each represent a hydrogen atom or a $C_{1-6}$ alkyl group; $R_3$ represent a hydroxy or an optionally protected hydroxy group in either the α- or β-configuration or an oxo group; $R_4$ represents a hydrogen atom or a halide or $R_3$ and $R_4$ together represent a carbon-carbon bond or an epoxy group in the β-configuration; $R_5$ represents a hydrogen atom or a halide in either the α- or β-configuration; and $R_{10}$ represent a fluoro-, chloro-, bromomethyl group or a 2'-fluoroethyl group, the method comprising:

A) reacting a steroidal carboxylic acid of formula (II)

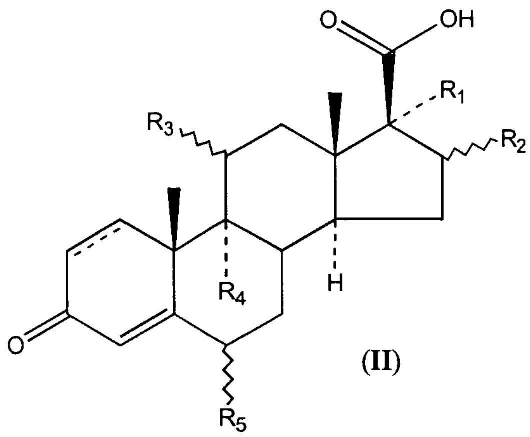

(II)

with a coupling enhancer of formula (A) or formula (B)

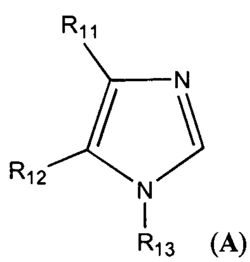

(A)

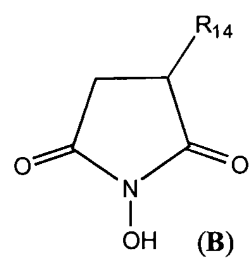

(B)

wherein $R_{11}$ and $R_{12}$ represent hydrogen atoms or cyano groups (C≡N); $R_{13}$ represent a hydrogen atom or a methyl group; and $R_{14}$ represent a hydrogen atom or a salt of a sulfonic acid;
B) reacting the *in situ* generated imidazolyl- or succinimidyl ester from step A) with a nucleophilic agent comprising sulfide; and
C) reacting the *in situ* product from step B) with an electrophilic agent.

**5.** The method of claim 4, wherein step A) is reacting the steroidal carboxylic acid with a coupling agent and the

coupling enhancer.

6. The method of any of the claims 4-5, wherein the nucleophilic agent is an alkoxide salt, a thioalkoxide salt, or a hydrated sulfide salt.

7. The method of any of claims 4-6, wherein step A), B) and C) are conducted in a one-pot synthesis without solvent changes and is conducted at room or elevated temperature.

8. The method of any of the claims 4-7, wherein the electrophilic agent is chlorofluoromethane or preferably bromofluoromethane.

9. The method of any of the claims 4-8, wherein the coupling agent is 1-ethyl-3-(3-dimethyl-aminopropyl) carbodiimide hydrochloride (EDC).

10. The method of any of the claims 4-9, wherein $R_{10}$ is a fluoromethyl group.

European Patent Office

# EUROPEAN SEARCH REPORT

Application Number
EP 03 00 7756

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | PHILLIPPS G H ET AL: "SYNTHESIS AND STRUCTURE-ACTIVITY RELATIONSHIPS IN A SERIES OF ANTIINFLAMMATORY CORTICOSTEROID ANALOGUES, HALOMETHYL ANDROSTANE-17BETA-CARBOTHIOATES AND-17BETA-CARBOSELENOATES" JOURNAL OF MEDICINAL CHEMISTRY, AMERICAN CHEMICAL SOCIETY. WASHINGTON, US, vol. 37, no. 22, 1 October 1994 (1994-10-01), pages 3717-3729, XP002025925 ISSN: 0022-2623 * page 3718 * scheme 3, compound 8 --- | 1 | C07J3/00 C07J31/00 |
| D,A | US 2002/133032 A1 (ABBOTT LABORARORIES) 19 September 2002 (2002-09-19) * page 6 * scheme 1 --- | 1-26 | |
| A | WO 97 24365 A (GLAXO GROUP LTD ;BIGGADIKE KEITH (GB); PROCOPIOU PANAYIOTIS ALEXAN) 10 July 1997 (1997-07-10) * claim 1 * --- | 1-26 | TECHNICAL FIELDS SEARCHED (Int.Cl.7) |
| | | | C07J |
| A | WO 02 08243 A (COOTE STEVEN JOHN ;ROBINSON JOHN MALCOLM (GB); GLAXO GROUP LTD (GB) 31 January 2002 (2002-01-31) * page 6-7 * ----- | 1-26 | |

The present search report has been drawn up for all claims

| Place of search | Date of completion of the search | Examiner |
|---|---|---|
| MUNICH | 25 June 2003 | Samsam Bakhtiary, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 03 00 7756

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2003

| Patent document cited in search report | Publication date | Patent family member(s) | Publication date |
|---|---|---|---|
| US 2002133032 A1 | 19-09-2002 | CA 2400919 A1 | 30-08-2001 |
| | | EP 1257531 A2 | 20-11-2002 |
| | | WO 0162722 A2 | 30-08-2001 |
| WO 9724365 A | 10-07-1997 | AT 194356 T | 15-07-2000 |
| | | AU 721865 B2 | 13-07-2000 |
| | | AU 1140997 A | 28-07-1997 |
| | | BG 102625 A | 30-04-1999 |
| | | BR 9612309 A | 13-07-1999 |
| | | CA 2241728 A1 | 10-07-1997 |
| | | CN 1209135 A | 24-02-1999 |
| | | CZ 9802074 A3 | 11-11-1998 |
| | | DE 69609199 D1 | 10-08-2000 |
| | | DE 69609199 T2 | 01-03-2001 |
| | | DK 876392 T3 | 06-11-2000 |
| | | EA 1401 B1 | 26-02-2001 |
| | | EE 9800227 A | 15-12-1998 |
| | | EP 0876392 A1 | 11-11-1998 |
| | | ES 2150150 T3 | 16-11-2000 |
| | | WO 9724365 A1 | 10-07-1997 |
| | | GR 3034564 T3 | 31-01-2001 |
| | | HK 1012193 A1 | 23-01-2001 |
| | | HU 9903707 A2 | 28-03-2000 |
| | | JP 2947944 B2 | 13-09-1999 |
| | | JP 11501675 T | 09-02-1999 |
| | | NO 983004 A | 26-08-1998 |
| | | NZ 324373 A | 28-10-1999 |
| | | PL 327629 A1 | 21-12-1998 |
| | | PT 876392 T | 29-12-2000 |
| | | SI 876392 T1 | 31-12-2000 |
| | | SK 89198 A3 | 10-03-1999 |
| | | TR 9801247 T2 | 23-11-1998 |
| | | US 6197761 B1 | 06-03-2001 |
| WO 0208243 A | 31-01-2002 | AU 7090601 A | 05-02-2002 |
| | | CZ 20023472 A3 | 16-04-2003 |
| | | EP 1301526 A1 | 16-04-2003 |
| | | WO 0208243 A1 | 31-01-2002 |
| | | NO 20025054 A | 05-11-2002 |

EPO FORM P0459

For more details about this annex : see Official Journal of the European Patent Office, No. 12/82